# EUROPEAN PATENT SPECIFICATION

(11) **EP 2 715 366 B1**
(45) Date of publication and mention of the grant of the patent: **19.07.2017**
(21) Application number: 12738256.2
(22) Date of filing: 03.04.2012
(51) Int. Cl.: G01N 33/574, G01N 33/50

(54) **BIOMARKERS FOR HEDGEHOG INHIBITOR THERAPY**
BIOMARKER FÜR HEDGEHOG-HEMMERTHERAPIE
BIOMARQUEURS POUR UNE THÉRAPIE PAR UN INHIBITEUR DE HEDGEHOG

(30) Priority: 02.06.2011 US 201161492603 P
(43) Date of publication of application: 09.04.2014
(73) Proprietor: Novartis AG, 4056 Basel (CH)
(72) Inventor: BANDARU, Raj, Winchester Massachusetts 01890 (US); HAIDER, Asifa, Succasunna New Jersey 07876 (US); ROBINSON, Douglas, Michael, Cambridge Massachusetts 02139 (US); ROSE, Kristine, Lynn, East Hanover New Jersey 07936 (US); SHARP, Thad, Tyson, Cambridge Massachusetts 02139 (US); SHOU, Yaping, Acton Massachusetts 01720 (US)
(74) Representative: Rudge, Sewkian
(86) International application number: PCT/US2012/031993
(87) International publication number: WO 2012/166241

(56) References cited:
- US-A1- 2009 012 018
- US-A1- 2011 046 211

## Description

### FIELD OF THE INVENTION

The present disclosure relates to a method of personalized therapy.

### BACKGROUND OF THE INVENTION

Hedgehog signaling is known to regulate a diverse range of biological processes, such as cellular proliferation, differentiation, and organ formation in a tissue specific and dose dependent manner. Normally, Hedgehog signaling is strictly controlled during cellular proliferation, differentiation and embryonic pattern formation. However, aberrant activity of the Hedgehog signaling pathway, due to mutations that constitutively activate the pathway, for instance, may have pathological consequences. By way of example, loss-of- function mutations of Patched are found in Gorlin's syndrome (a hereditary syndrome with high risk of skin and brain cancers, also known as Basal Cell Nevus Syndrome (BCNS)); and gain-of- function mutations of Smo and GIi are linked to basal cell carcinoma and glioblastoma. Basal cell carcinoma (BCC) is the most common form of skin cancer, affecting more than 90,000 Americans each year.

Constitutive activation of Hedgehog has been found to promote tumorigenesis in BCC, medulloblastoma (the most common childhood brain tumor), rhabdomyosarcoma, pancreatic cancer, small cell lung cancer, prostate cancer and breast cancer. Besides the roles in tumorigenesis, Hedgehog signaling is also implicated in the metastasis of prostate cancer. Hedgehog signaling may be involved in many additional types of tumor types and such links are expected to continue to be discovered; this is an area of active research in many cancer centers around the world.

US 2009/012018 describes compositions and methods comprising Wnt and Hedgehog pathway inhibitors for inhibition of signal transduction pathways in cancer cells. US2011/046211 describes the use of a combination of a hedgehog inhibitor with chemotherapy and/ or radiation.

### SUMMARY OF THE INVENTION

The present invention provides:
- a method of analyzing a biological sample of a subject with cancer, comprising determining a level of expression of all of the biomarkers GLI-1, OTX-2, SHROOM2, PDLIM3, and SPHK1 in the biological sample taken from the subject, wherein the level of expression of the biomarkers in comparison to a control provide a diagnostic indicator of whether the subject has an increased likelihood of response to a methyl-4'-trifluoromethoxy-biphenyl-3-carboxylic acid [6-(cis-2,6-dimethyl-morpholin-4-yl)-pyridin-3-yl]-amide or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol;
- a method of selecting a subject having cancer for treatment with a Hedgehog signaling inhibitor, the method comprising determining the level of expression of at least five biomarkers selected from the group consisting of GLI-1, OTX-2, SHROOM2, PDLIM3, SPHK1, SFRP1, APBA2 and SPATA20, in a biological sample derived from the subject, thereby to predict an increased likelihood of response to a Hedgehog signaling inhibitor;
- a method of selecting a subject having cancer for treatment with methyl-4'-trifluoromethoxy-biphenyl-3-carboxylic acid [6-(cis-2,6-dimethyl-morpholin-4-yl)-pyridin-3-yl]-amide or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol, the method comprising determining the level of SHROOM2 in a biological sample derived from the subject, thereby to predict an increased likelihood of response to methyl-4'-trifluoromethoxy-biphenyl-3-carboxylic acid [6-(cis-2,6-dimethyl-morpholin-4-yl)-pyridin-3-yl]-amide or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol;
- a method of selecting a subject having cancer for treatment with methyl-4'-trifluoromethoxy-biphenyl-3-carboxylic acid [6-(cis-2,6-dimethyl-morpholin-4-yl)-pyridin-3-yl]-amide or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol, the method comprising determining the level of SPHK1 in a biological sample derived from the subject, thereby to predict an increased likelihood of response to methyl-4'-trifluoromethoxy-biphenyl-3-carboxylic acid [6-(cis-2,6-dimethyl-morpholin-4-yl)-pyridin-3-yl]-amide or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol;
- a Hedgehog signaling inhibitor, wherein the inhibitor is Jervine, GANT61, purmorphamine, SAG, SANT-2, tomatidine, zerumbone, GDC-0449, XL139, IPI926, IPI609 (IPI269609), BMS-833923/XL139, TAK-441, PF-04449913, methyl-4'-trifluoromethoxy-biphenyl-3-carboxylic acid [6-(cis-2,6-dimethyl-morpholin-4-yl)-pyridin-3-yl]-amide, or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol, for use in a method of treating cancer in a patient in need thereof, comprising selectively administering the Hedgehog signaling inhibitor to the patient on the basis of the patient has an elevated level of expression of the biomarkers GLI-1, SHROOM2, PDLIM3, and SPHK1 and a decreased level of expression of the biomarker OTX-2.

The present invention is based on the finding that particular biomarkers can be used to select individuals having cancer who are likely to respond to treatment with an inhibitor of the Hedgehog signaling pathway. Specifically, it was found the level of expression of a biomarker listed in Table 1, e.g., the mRNA expression of a biomarker listed in Table 1 and/or the increased presence or reduction in the amount of a protein product encoded by a biomarker listed in Table 1 in a sample from an individual having cancer compared to a control, can be used to predict whether that individual will respond to treatment with an inhibitor of the Hedgehog signaling pathway.

In one aspect, the invention includes a method of selecting a subject having cancer for treatment with a Hedgehog signaling inhibitor, the method including determining the level of expression of at least five biomarkers listed in Table 1, such as SHROOM 2 or SPHK1, in a biological sample derived from the subject, thereby to predict an increased likelihood of response to a Hedgehog signaling inhibitor. In one embodiment, the invention includes selecting at least five, at least six, at least seven, or at least eight biomarkers in Table 1. In the method of the invention, the hedgehog signaling inhibitor is cyclopamine, Jervine, GANT61, purmorphamine, SAG, SANT-2, tomatidine, zerumbone, GDC-0449; XL139, IPI926, IPI609 (IPI269609), or BMS-833923/XL139, or derivatives thereof. In one embodiment, the Hedgehog signaling inhibitor is methyl-4'-trifluoromethoxy-biphenyl-3-carboxylic acid [6-(cis-2,6-dimethyl-morpholin-4-yl)-pyridin-3-yl]-amide (Compound I) or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol Compound II. In the method of the invention, the cancer can be BCC, Chronic myeloid leukemia (CML), bone sarcoma, soft tissue sarcoma, medulloblastoma , rhabdomyosarcoma, pancreatic cancer, small cell lung cancer, prostate cancer, Gorlin syndrome, gastro-esophageal cancer, myeloproliferative neoplasia and acute leukemias or breast cancer. In one embodiment, the biological sample is a tumor sample such as a fresh frozen sample or a formalin fixed paraffin-embedded tissue sample.

There is also disclosed a method of selecting a subject having a tumor for treatment with a hedgehog signaling inhibitor, the method comprising determining the level of expression of at least one biomarker listed in Table 1 in a biological sample derived from the subject having medullablastoma thereby to predict an increased likelihood of response to methyl-4'-trifluoromethoxy-biphenyl-3-carboxylic acid [6-(cis-2,6-dimethyl-morpholin-4-yl)-pyridin-3-yl]-amide.

The level of expression using the methods described herein can include determining the level of expression of mRNA or determining protein level.

In another aspect, there is disclosed a method of selecting a subject having cancer for treatment with a hedgehog signaling inhibitor, the method including determining the level of expression of a Hedgehog signaling biomarker listed in Table 1 in a biological sample derived from the subject having cancer; determining the level of mRNA of one or more reference/normalized genes listed in Table 2; and normalizing the expression level of the hedgehog signaling biomarker to the reference gene.

In another aspect, a kit for determining if a tumor is responsive to treatment with a hedgehog signaling inhibitor comprising providing one or more probes or primers for detecting the expression level of at least one, at least two, at least three, at least four, at least five, at least six, at least seven, or at least eight biomarkers listed in Table 1 is disclosed. The kit can include a plurality of agents for determining the level of one or more biomarkers listed in Table 1 and optionally also include agents for determining the level of expression of one or more biomarkers listed in Table 2 and instructions for use.

In one aspect, the kit can be a kit for predicting whether a subject with cancer would benefit from treatment with a hedgehog signaling inhibitor, the kit including: a plurality of agents for determining the mRNA expression level of one or more biomarkers identified in Table 1; and means for analyzing the expression and generating a score to predict whether a patient would benefit from treatment with a hedgehog signaling inhibitor. The agents for determining mRNA expression can include an array of polynucleotides complementary to one or more biomarkers identified in Table 1. The agents for measuring mRNA expression include a plurality of PCR probes and/or primers for qRT-PCR.

In yet another aspect, a microarray comprising polynucleotide probes complementary and hybrdizable to at least one biomarker listed in Table 1 is disclosed.

In yet another aspect, composition comprising a plurality of isolated nucleic acid sequences, wherein each isolated nucleic acid sequence hybridizes to at least one RNA products selected from the following genes GLI-1, OTX-2, SHROOM2, PDLIM3, SPHK1, SFRP1, APBA2 and SPATA20, wherein the composition is used to measure the level of mRNA expression of the genes, is also disclosed.

In still yet another aspect, there is also disclosed a computer product for selecting a subject having or suspected of having medullablastoma for treatment with a SMO inhibitor drug: means for receiving data corresponding to the expression level of at least one gene in a sample from the subject listed in Table 1; means for generating an expression value for each gene; and means for generating a score based on inputting the expression value into a database comprising a reference expression profile associated with selection, wherein score predicts whether the subject would benefit from treatment with a SMO inhibitor. The computer product can be used in any of the methods described above.

A "biomarker" is a molecule useful as an indicator of a biologic state in a subject. With reference to the present subject matter, the biomarkers disclosed herein can be molecules that exhibit a change in expression to predict whether a subject would benefit from receiving a treatment with a hedgehog signaling inhibitor, e.g., a SMO inhibitor.

"Hedgehog" refers generically to any of the mammalian homologs of the Drosophila hedgehog protein, and includes at least Sonic hedgehog (SHedgehog), Desert hedgehog (DHedgehog) and Indian hedgehog (Hedgehog).

"Hedgehog signaling pathway" as used herein refers to the signaling cascade mediated by (or downstream of) hedgehog and its receptors which results in changes of gene expression and other phenotypic changes typical of hedgehog activity. Activation of the signaling pathway can be due to a Hedgehog signaling component that positively affects the transmission of the Hedgehog signal, i.e., stimulates downstream biological events when Hedgehog is present. Examples of such components are Hedgehog, Ptch, Smo, and Gli. Hedgehog positive (Hh+) tumors are those tumors having genetic alterations that results in constitutive hedgehog pathway activation and this activation of Hedgehog appears to be the major driver for tumor formation and growth.

### DETAILED DESCRIPTION OF THE INVENTION

There is an increasing body of evidence that suggests a patient's genetic profile can be determinative to a patient's responsiveness to a therapeutic treatment. Given the numerous therapies available to treat cancer, a determination of the genetic factors that influence, for example, response to a particular drug, could be used to provide a patient with a personalized treatment regime. Such personalized treatment regimes offer the potential to maximize therapeutic benefit to the patient while minimizing related side effects that can be associated with alternative treatment regimes. Thus, there is a need to identify factors which can be used to predict whether a patient is likely to respond to a particular therapy.

To maximize the potential clinical benefit of a patient receiving a Hedgehog signaling inhibitor it is important to be able to select those patients who have tumors that have an activated Hedgehog signaling pathway. The methods described herein are based, in part, upon the identification of a single or a plurality of biomarkers listed in Table 1, which can be used to determine a patient's likelihood of benefiting from treatment with a Hedgehog signaling inhibitor such as a SMO inhibitor.

The biomarkers of the disclosure were purposefully optimized for use in Formalin-Fixed, Paraffin-Embedded tissue (FFPE) specimens to make it applicable for routine clinical testing.

### Hedgehog signaling inhibitors

Hedgehog signaling inhibitors are agents known to inhibit the Hedgehog signaling pathway. Such agents can be agents that inhibit aberrant growth states resulting from phenotypes such as loss-of- function mutations in Ptch or Sufu, or gain-of-function mutations in Hedgehog, Smoothened, or Gli. Hedgehog inhibitors are known in the art, and include for example small molecule compounds, small peptides, antibodies, antisense oligonucleotides, siRNAs, and the like.

In some embodiments, the hedgehog signaling inhibitor is a small molecule compound. In some embodiments, the hedgehog signaling inhibitor is a cyclopamine or derivative thereof. In some embodiments, the hedgehog signaling inhibitor is Jervine, GANT61, purmorphamine, SAG, SANT-2, tomatidine, zerumbone, or derivatives thereof. In some embodiments, the hedgehog signaling inhibitor is GDC-0449 (available from Genentech and/or Curis); XL139, IPI926 (available from Infinity Pharmaceuticals), IPI609 (IPI269609), BMS-833923/XL139 (available from Bristol-Myers Squibb and/or Exelixis, TAK-441 (Millennium) or PF-04449913 (Pfizer).

Additional hedgehog signaling inhibitors are described in PCT/US2010/038568, PCT/US2010/044168, PCT/US2009/061573, PCT/US2009/063696PCT/US2009/039065.

The hedgehog signaling inhibitors described herein can be the agents themselves, pharmaceutically acceptable salts thereof, pharmaceutically acceptable esters thereof, as well as steroisomers, enantiomers, racemic mixtures, and the like. In one embodiment, the hedgehog signaling inhibitor is a SMO inhibitor such as 2-methyl-4'-trifluoromethoxy-biphenyl-3-carboxylic acid [6-(cis-2,6-dimethyl-morpholin-4-yl)-pyridin-3-yl]-amide, also known as N-[6-(cis-2,6-dimethylmorpholin-4-yl)pyridine-3-yl]-2-methyl-4'-(trifluoromethoxy)[1,1'-biphenyl]-3-carboxamide, which is disclosed in International Patent Application Nos. WO 2007/131201 and in WO 2008/154259. In another embodiment the hedgehog signaling inhibitor is 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol, disclosed in WO 2010/007120.

The hedgehog signaling inhibitors includes a compound of Formula I: 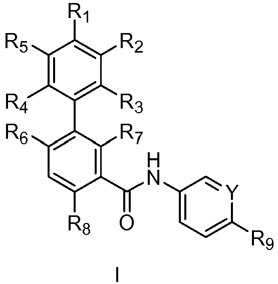 in which
Y is selected from N and CR₁₀; wherein R₁₀ is selected from hydrogen, halo, C₁₋₆alkyl, halosubstituted-C₁₋₆alkyl, C₁₋₆alkoxy, halosubstituted-C₁₋₆alkoxy and -OXNR₁₀ₐR_{10b}; wherein R₁₀ₐ and R_{10b} are independently selected from hydrogen and C₁₋₆alkyl;
R₁ is selected from cyano, halo, C₁₋₆alkyl, halosubstituted-C₁₋₆alkyl, C₁₋₆alkoxy, halosubstituted-C₁₋₆alkoxy, C₆₋₁₀aryl, dimethyl-amino, C₁₋₆alkyl-sulfanyl and C₃₋₈heterocycloalkyl optionally substituted with up to 2 C₁₋₆alkyl radicals;
R₂ and R₅ are independently selected from hydrogen, cyano, halo, C₁₋₆alkyl, halosubstituted-C₁₋₆alkyl, C₁₋₆alkoxy, halosubstituted-C₁₋₆alkoxy and dimethylamino;
R₃ and R₄ are independently selected from hydrogen, halo, cyano, C₁₋₆alkyl, halosubstituted-C₁₋₆alkyl, C₁₋₆alkoxy and halosubstituted-C₁₋₆alkoxy; or either R₁ and R₂ or R₁ and R₅ together with the phenyl to which they are both attached form C₅₋₁₀heteroaryl;
R₆ and R₇ are independently selected from hydrogen, C₁₋₆alkyl, halosubstituted-C₁₋₆alkyl, C₁₋₆alkoxy and halosubstituted-C₁₋₆alkoxy; with the proviso that R₆ and R₇ are not both hydrogen;
R₈ is selected from hydrogen, halo, C₁₋₆alkyl, halosubstituted-C₁₋₆alkyl, C₁₋₆alkoxy and halosubstituted-C₁₋₆alkoxy;
R₉ is selected from -S(O)₂R₁₁ and -R₁₁; wherein R₁₁ is selected from aryl, heteroaryl, cycloalkyl and heterocycloalkyl;
wherein said aryl, heteroaryl, cycloalkyl and heterocycloalkyl of R₉ can be optionally substituted with 1 to 3 radicals independently selected from C₁₋₆alkyl, halosubstituted-C₁₋₆alkyl, C₁₋₆alkoxy, halosubstituted-C₁₋₆alkoxy, C₆₋₁₀aryl-C₀₋₄alkyl, C₅₋₁₀heteroaryl-C₀₋₄alkyl, C₃₋₁₂cycloalkyl and C₃₋₈heterocycloalkyl;
wherein said aryl-alkyl substituent of R₉ is optionally substituted with 1 to 3 radicals independently selected from halo, C₁₋₆alkyl, halosubstituted-C₁₋₆alkyl, C₁₋₆alkoxy, halosubstituted-C₁₋₆alkoxy and methyl-piperazinyl;; and a pharmaceutically acceptable salts thereof.

There is also disclosed a hedgehog signaling inhibitors of Formula I(a): 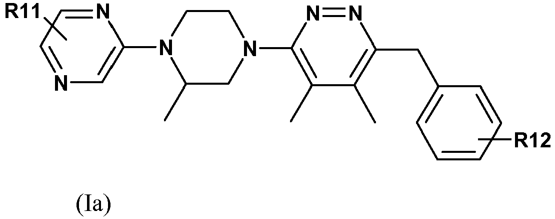 or a pharmaceutically acceptable salt thereof, wherein
R11 is C₁₋₈ alkyl, C₂₋₈ alkenyl, C₃₋₁₄ cycloalkyl, a C₆₋₁₄ aryl group, a 5-14 membered heteroaryl group, a 3-14 membered cycloheteroalkyl group, C₁₋₈ alkoxy, halo, NR13R14, C(O)OR13, C(O)NR13R14, C₁₋₈haloalkyl, formyl, carbalkoxy, C₁₋₈alkylOH, C(O)R13, SO₂R13, C(O)NHC₁₋₈alkylR13, NR13R14, SO₂NR13R14, OCF₃, NHC(O)R13, CH₂OC(O)NR13R14, CH₂NR13R14, NHC(O)OR13, NHC(O)NR13R14, CH₂NHSO₂R13, CH₂NHC(O)OR13, OC(O)R13, or NHC(O)R13, which may be substituted or unsubstituted;
R12 is H, C₁₋₈ alkyl, a C₆₋₁₄ aryl group, C₁₋₈ haloalkyl, C₁₋₈ alkoxy, halo, NH₂, CN, OCF₃, OH, C(O)NR13R14, C(O)R13, NR13R14, NHC(O)R13, SO₂R13, SO₂NR13R14;
R13 and R14 are independently H, C₁₋₈ alkyl, C₂₋₈ alkenyl, C₃₋₁₄ cycloalkyl, a C₆₋₁₄ aryl group, a 5-14 membered heteroaryl group, a 3-14 membered cycloheteroalkyl group, C₁₋₈haloalkyl, C₁₋₈ alkylOH, C₁₋₈alkoxy, or R13 and R14 on one atom can form a heteroatom containing ring; and
Wherein R11, R13, and R14 can be unsubstituted or substituted by one or more of C₁₋₈ alkyl, C₃₋₁₄ cycloalkyl, a C₆₋₁₄ aryl group, a 5-14 membered heteroaryl group, a 3-14 membered cycloheteroalkyl group, C₁₋₈ alkylOH, OH, oxo, C₁₋₈ haloalkyl, carboxC₁₋₈ alkyl, or SO₂C₁₋₈alkyl, halo, -OCH₃, -OCF₃, -OH, -NH₂.

### Biomarker

The biomarker(s) of the disclosure includes one or more genes listed in Table 1 or their gene products. By analyzing the expression level of one or more biomarkers identified in Table 1 it is possible to select individuals having cancers in which the hedgehog pathway is activated and who thus are likely to respond to treatment with an inhibitor of the Hedgehog signaling pathway, e.g., a Smoothened (SMO) antagonist.

The biomarkers of the disclosure include GLI-1, OTX-2, SHROOM2, PDLIM3, SPHK1, SFRP1, APBA2 and SPATA20. GLI-1, SHROOM2, PDLIM3, SPHK1, SFRP1, and APBA2 are upregulated (increase in mRNA expression) while OTX-2 and SPATA20 are downregulated (decrease in mRNA expression). In one example, the expression profile can be a set of values representing mRNA levels of one or more of the following genes GLI-1, OTX-2, SHROOM2, PDLIM3, SPHK1, SFRP1, APBA2 and SPATA20. In another example, the expression profile can be a set of values representing mRNA levels of one or more of the following genes GLI-1, OTX-2, SHROOM2, PDLIM3 and SPHK1. In yet another example, the expression profile can include a set of values representing one or more proteins or polypeptides encoded by GLI-1, OTX-2, SHROOM2, PDLIM3, SPHK1, SFRP1, APBA2 and SPATA20. The phrase "mRNA expression" can mean an increase or decrease in the amount of mRNA expression in a sample from an individual having cancer compared to a control sample. Typically, an increase or decrease in mRNA expression means a 1.5 fold, or more (such as a 2, 3, 4, or 5 fold), difference in expression as compared with a control (e.g., normal level as determined from a control).

### Preparation of Samples

Any appropriate test sample of cells taken from an individual having a proliferative disease can be used. Generally, the test sample of cells or tissue sample will be obtained from the subject with cancer by biopsy or surgical resection. A sample of cells, tissue, or fluid may be removed by needle aspiration biopsy. For this, a fine needle attached to a syringe is inserted through the skin and into the tissue of interest. The needle is typically guided to the region of interest using ultrasound or computed tomography (CT) imaging. Once the needle is inserted into the tissue, a vacuum is created with the syringe such that cells or fluid may be sucked through the needle and collected in the syringe. A sample of cells or tissue may also be removed by incisional or core biopsy. For this, a cone, a cylinder, or a tiny bit of tissue is removed from the region of interest. CT imaging, ultrasound, or an endoscope is generally used to guide this type of biopsy. More particularly, the entire cancerous lesion may be removed by excisional biopsy or surgical resection. The test sample is typically a sample of cells removed as part of surgical resection.

The test sample of, for example tissue, may also be stored in, e.g., RNAlater (Ambion; Austin Tex.) or flash frozen and stored at -80°C. for later use. The biopsied tissue sample may also be fixed with a fixative, such as formaldehyde, paraformaldehyde, or acetic acid/ethanol. The fixed tissue sample may be embedded in wax (paraffin) or a plastic resin. The embedded tissue sample (or frozen tissue sample) may be cut into thin sections. RNA or protein may also be extracted from a fixed or wax-embedded tissue sample or a frozen tissue sample. Once a sample of cells or sample of tissue is removed from the subject with cancer, it may be processed for the isolation of RNA or protein using techniques well known in the art and as described below.

An example of extraction of RNA from a biopsy taken from a patient with cancers can include, for example, guanidium thiocyanate lysis followed by CsCl centrifugation (Chirgwin, et al., Biochemistry 18:5294-5299, 1979). RNA from single cells may be obtained as described in methods for preparing cDNA libraries from single cells (see, e.g., Dulac, Curr. Top. Dev. Biol. 36:245, 1998; Jena, et al., J. Immunol. Methods 190:199, 1996). In one embodiment, the RNA population may be enriched for sequences of interest, as detailed in Tables 1 and 2. Enrichment may be accomplished, for example, by random hexamers and primer-specific cDNA synthesis, or multiple rounds of linear amplification based on cDNA synthesis and template-directed in vitro transcription (see, e.g., Wang, et al., Proc. Natl. Acad. Sci. USA 86:9717, 1989; Dulac, et al., supra; Jena, et al., supra). Other methods of isolating RNA from a sample are known in the art and include Trizol (Invitrogen), Guanidinium thiocyanate-phenol-chloroform extraction, PureLink Micro-to- Midi Total RNA Purification System (invitrogen), RNeasy kit (Qiagen), Oligotex kit (Qiagen), PureYield™ RNA Midiprep (Promega), PolyATtract System 1000 (Promega), Maxwell(R) 16 System (Promega), SV Total RNA Isolation (Promega), ToTALLY RNA™ Kit (Ambion), Poiy(A)Purist™ Kit (Ambion) and any other methods. Methods for extracting and analysing an RNA sample are disclosed in Molecular Cloning, A Laboratory Manual (Sambrook and Russell (ed.), 3rd edition (2001), Cold Spring Harbor Laboratory Press, Cold Spring Harbor, New York, USA.

The Hedgehog expression profile can be performed on a biopsy taken from a subject such as fresh tissue, frozen tissue, tissue processed in formalin (FFPE) or other fixatives. In particular, where the sample is an FFPE sample, RNA is extracted from FFPE sections using the Qiagen RNeasy FFPE extraction kit (Qiagen), and reverse transcribed to cDNA using random hexamers and ABI's High Capacity cDNA archive kit (Applied Biosystems, Foster City, CA).

The subject with a tumor or cancer will generally be a mammalian subject such as a primate. In an exemplary embodiment, the subject is a human. As used herein the terms patient and subject are synonymous.

Any cancer or tumor can be screened according to the methods of the invention and include, but are not limited to, colon cancer, lung cancer, pancreatic cancer, gastric cancer, prostate cancer, and hepatocellular carcinoma, basal cell carcinoma, breast cancer, bone sarcoma, soft tissue sarcoma, chronic myeloid leukemia, acute myeloid leukemia, hematological cancer, medulloblastoma, rhabdomyosaracoma, neuroblastoma, pancreatic cancer, breast carcinoma, meningioma, glioblastoma, astrocytoma, melanoma, stomach cancer, esophageal cancer, biliary tract cancer, prostate cancer, small cell lung cancer, non-small cell lung cancer, glial cell cancer, multiple myeloma, colon cancer, neuroectodermal tumor, neuroendocrine tumor, mastocytoma and Gorlin syndrome, glioma, colorectal cancer, GIST, gastro-esophageal cancer, myeloproliferative neoplasia and an acute leukemia.

### Detection of expression of the biomarker

In one example, the method includes determining expression of one or more of the genes GLI-1, OTX-2, SHROOM2, PDLIM3, SPHK1, SFRP1, APBA2 and SPATA20. The gene sequences of interest can be detected using agents that can be used to specifically detect the gene, for example, RNA transcribed from the gene or polypeptides encoded by the gene.

In one embodiment, the method includes: providing a nucleic acid probe comprising a nucleotide sequence, for example, at least 10, 15, 25 or 40 nucleotides, and up to all or nearly all of the coding sequence which is complementary to a portion of the coding sequence of a nucleic acid sequence of GLI-1, OTX-2, SHROOM2, PDLIM3, SPHK1, SFRP1, APBA2 and SPATA20; obtaining a tissue sample from a mammal having a cancerous cell; contacting the nucleic acid probe under stringent conditions with RNA obtained from a biopsy taken from a patient with medulablastoma (e.g., in a Northern blot, in situ hybridization assay, PCR etc); and determining the amount of hybridization of the probe with RNA. Nucleic acids may be labeled during or after enrichment and/or amplification of RNAs.

The biomarkers GLI-1, OTX-2, SHROOM2, PDLIM3, SPHK1, SFRP1, APBA2 and SPATA20 are intended to also include naturally occurring sequences including allelic variants and other family members. The biomarkers of the disclosure also include sequences that are complementary to those listed sequences resulting from the degeneracy of the code and also sequences that are sufficiently homologous and sequences which hybridize under stringent conditions to the genes of the dislcosure.

Conditions for hybridization are known to those skilled in the art and can be found in Current Protocols in Molecular Biology, John Wiley and Sons, N.Y. (1989), 6.3.1-6.3.6. A preferred, non-limiting example of highly stringent hybridization conditions are hybridization in 6 X sodium chloride/sodium citrate (SSC) at about 45 degrees centigrade followed by one or more washes in 0.2 X SSC, 0.1 percent SDS at 50-65 degrees centigrade. By "sufficiently homologous" it is meant a amino acid or nucleotide sequence of a biomarker which contains a sufficient or minimum number of identical or equivalent (e.g., an amino acid residue which has a similar side chain) amino acid residues or nucleotides to a second amino acid or nucleotide sequence such that the first and second amino acid or nucleotide sequences share common structural domains or motifs and/or a common functional activity. For example, amino acid or nucleotide sequences which share common structural domains have at least about 50 percent homology, at least about 60 percent homology, at least about 70 percent, at least about 80 percent, and at least about 90-95 percent homology across the amino acid sequences of the domains are defined herein as sufficiently homologous. Furthermore, amino acid or nucleotide sequences at least about 50 percent homology, at least about 60-70 percent homology, at least about 70-80 percent, at least about 80-90 percent, and at least about 90-95 percent and share a common functional activity are defined herein as sufficiently homologous.

The comparison of sequences and determination of percent homology between two sequences can be accomplished using a mathematical algorithim. A preferred, non-limiting example of a mathematical algorithim utilized for the comparison of sequences is the algorithm of Karlin and Altschul (1990) Proc. Natl. Acad. Sci. USA 87:2264-68, modified as in Karlin and Altschul (1993) Proc. Natl. Acad. Sci. USA 90:5873-77. Such an algorithm is incorporated into the NBLAST and XBLAST programs (version 2.0) of Altschul, et al. (1990) J. MoI. Biol. 215:403-10. BLAST nucleotide searches can be performed with the NBLAST program, score=100, wordlength=12 to obtain nucleotide sequences homologous to TRL nucleic acid molecules of the disclosure. BLAST protein searches can be performed with the XBLAST program, score=50, wordlength=3 to obtain amino acid sequences homologous to the protein sequences encoded by the genes/oligonucleotides listed in Table 1, 2 and/or Table 3. To obtain gapped alignments for comparison purposes, Gapped BLAST can be utilized as described in Altschul et al., (1997) Nucleic Acids Research 25(17):3389-3402. When utilizing BLAST and Gapped BLAST programs, the default parameters of the respective programs (e.g., XBLAST and NBLAST) can be used. See http://www.ncbi.nlm.nih.gov. Another preferred, non-limiting example of a mathematical algorithim utilized for the comparison of sequences is the ALIGN algorithm of Myers and Miller, CABIOS (1989). When utilizing the ALIGN program for comparing amino acid sequences, a PAM1 20 weight residue table, a gap length penalty of 12, and a gap penalty of 4 can be used.

The present disclosure includes measuring the expression of one or more genes GLI-1, OTX-2, SHROOM2, PDLIM3, SPHK1, SFRP1, APBA2 and SPATA20 in a tumor biopsy taken from a subject suffering from cancer due to hedgehog pathway activation. The expression levels can be analyzed and used to generate a score which can be used to differentiate those patients having a tumor exhibiting hedgehog pathway activation versus those who do not.

The method of the disclosure includes measuring any one of GLI-1, OTX-2, SHROOM2, PDLIM3, SPHK1, SFRP1, APBA2 and SPATA20 listed in Table 1. The method disclosed herein also includes measuring at least two, at least three, at least four, at least five, at least six, at least seven, or at least eight genes from Table 1.

In one example, the level of expression of one gene, e.g., GLI-1, from Table 1 is measured. In another example, the level of expression of two genes, e.g., GLI-1, OTX-2, from Table 1 is measured. In yet another example, the level of expression of three genes GLI-1, OTX-2, and SHROOM2 from Table 1 is measured. In yet another example, the level of expression of four genes GLI-1, OTX-2, SHROOM2 or PDLIM3 from Table 1 is measured. In yet another example, the level of expression of five genes GLI-1, OTX-2, SHROOM2, PDLIM3 and SPHK1 from Table 1 is measured.

**Table 1**

| **Gene Name** | **Accession # Uni Gene ID** |
|---|---|
| GLI-1 | UGID:2139596 |
| OTX-2 | UGID:178376 |
| SHROOM2 | UGID:1782725 |
| PDLIM3 | UGID:237739 |
| SPHK1 | UGID:139253 |
| SFRP1 | UGID:164788 |
| APBA2 | UGID:2087123 |
| SPATA20 | UGID:143131 |

The biomarkers of the disclosure also include any combination of genes identified in Table 1 whose level of expression or gene product serves as a predictive biomarker. The biomarkers of the disclosure, including their gene sequence, are known in the art (as provided above) or, for example, for GLI-1 ((GLI family zinc finger 1; Nature 332:371-374(1988)), for OTX-2 (Orthodenticle homeobox 2; EBO J. 12:2735-2747(1993)), for SHROOM2 (Shroom family member 2; Hum. Mol. Genet. 4:373-382(1995)), for PDLIM3 (PDZ and LIM domain 3; J. Cell Biol. 139:507-515(1997)), for SPHK1 (sphingosine kinase 1; Gene 251:19-26(2000)), for SFRP1 (secreted frizzled-related protein 1; Proc. Natl. Acad. Sci. U.S.A. 94:6770-6775(1997)), for APBA2 (amyloid beta (A4) precursor protein-binding, family A; J. Biol. Chem. 272:31459-31464(1997)), for SPATA20 (spermatogenesis associated 20; Proc. Natl. Acad. Sci. U.S.A. 99 (26), 16899-16903 (2002)).

In the method described herein the level of expression of one or more genes as described in Table 1 is measured and analyzed and compared to a control. The control for comparison can be determined by one skilled in the art. In one example, the control is determined by choosing a value that serves as a cut-off value. For example, the value can be a value that differentiates between e.g., those test samples that have hedgehog activation (hedgehog +) from those that do not show hedgehog activation (hedgehog -). In another example, the gene expression profile of a biomarker of the disclosure is compared to a control (presence of expression of the biomarker in a sample taken from a healthy person or a tumor that is hedgehog-activated).

In a particular embodiment of the disclosure, the control is predetermined and a score is generated which can be used to select those subjects having a tumor due to hedgehog pathway activation. The expression threshold can be used to select for those individuals who have will respond to a hedgehog signaling inhibitor.

In another example, the control can include one or more normalized genes (such as described below) which are genes that exhibit a relatively constant level of gene expression. The normalized genes correct for (normalize away) both differences in the amount of RNA assayed and variability in the quality of the RNA used. Therefore, the assay typically measures and incorporates the expression of certain normalizing genes such as those normalized genes shown in Table 2.

**Table 2**

| **Gene Name** | **Accession #** |
|---|---|
| HUWE1 | UGID: 150675 |
| LARP1 | UGID: 179374 |
| SOD1 | UGID: 238951 |
| YME1L1 | UGID: 714304 |

In one of the methods disclosed herein, the expression of one or more of the genes of Table 1 (e.g., two, three, four, five, six, seven, or eight genes) is measured and typically will be converted into an expression value after normalization by the expression level of the single control gene or the average of 4 or 3 or 2 control genes described in Table 2. These expression values then will be used to generate a score which is then compared against a cut-off to select which subjects have a Hedgehog-activated tumor and therefore are likely to benefit from treatment with a Hedgehog signaling inhibitor. In one example, the mRNA level of at last two, three, four or all five genes of GLI-1, OTX-2, SHROOM2, PDLIM3 and SPHK1 are measured and the mRNA values are converted into an expression value after normalization by the expression level of the a control gene or the average of 4 or 3 or 2 control genes described in Table 2. The normalized genes of the disclosure UBA and WWE domain containing 1 (HUWEI), La ribonucleoprotein domain family, member 1 (LARP1), Superoxide dismutase 1, soluble (SOD1), of the disclosure, YME1-like 1 (YME1L1) including their gene sequence, are known in the art, for example, gene sequence accession numbers as provided by the NCBI are provided above.

The biomarkers of the disclosure can be measured using any method known in the art such as reverse Transcriptase PCR (RT-PCR). The method includes isolating mRNA using any technique known in the art and described above, e.g., by using a purification kit, buffer set and protease from commercial manufacturers, such as Qiagen. The reverse transcription step is typically primed using specific primers, random hexamers, or oligo-dT primers, depending on the circumstances and the goal of expression profiling and the cDNA derived can then be used as a template in the subsequent PCR reaction. TaqMan(R) RT-PCR can then be performed using , e.g., commercially available equipment.

The isolated mRNA can then be further analyzed using any method known in the art such as microarray analysis, quantitative ('real-time') PCR, northern blotting, and nuclease protection assay. In one example, real time quantitative PCR is used which measures PCR product accumulation through a dual-labeled fluorigenic probe (e.g., using TaqMan(R) probe). Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al, Genome Research 6:986-994 (1996). In a real time PCR assay a positive reaction is detected by accumulation of a fluorescent signal. The Ct (cycle threshold) is defined as the number of cycles required for the fluorescent signal to cross the threshold (i.e. exceeds background level). Ct levels are inversely proportional to the amount of target nucleic acid in the sample (i.e. the lower the Ct level the greater the amount of target nucleic acid in the sample). Most real time assays undergo 40 cycles of amplification.

In another example, microarrays are used which include one or more probes corresponding to one or more of genes GLI-1, OTX-2, SHROOM2, PDLIM3, SPHK1, SFRP1, APBA2 and SPATA20. Use of a microarray results in the production of hybridization patterns of labeled target nucleic acids on the array surface. The resultant hybridization patterns of labeled nucleic acids may be visualized or detected in a variety of ways, with the particular manner of detection selected based on the particular label of the target nucleic acid. Representative detection means include scintillation counting, autoradiography, fluorescence measurement, calorimetric measurement, light emission measurement, light scattering, and the like.

In another example, a TaqMan® Low Density Array (TLDA) card can be used which can include one or more probes corresponding to one or more of genes GLI-1, OTX-2, SHROOM2, PDLIM3, SPHK1, SFRP1, APBA2 and SPATA20. This method uses a microfluidic card that performs simultaneous real time PCR reactions.

In one example, the method of detection utilizes an array scanner that is commercially available (Affymetrix, Santa Clara, Calif.), for example, the 417.TM. Arrayer, the 418.TM. Array Scanner, or the Agilent GeneArray.TM. Scanner. This scanner is controlled from a system computer with an interface and easy-to-use software tools. The output may be directly imported into or directly read by a variety of software applications. Scanning devices are described in, for example, U.S. Pat. Nos. 5,143,854 and 5,424,186.

### Defecting expression of the biomarker gene product

Detecting for the presence of a protein product encoded by one or more of GLI-1, OTX-2, SHROOM2, PDLIM3, SPHK1, SFRP1, APBA2 and SPATA20 can be done by using any appropriate method known in the art. For example, an agent of interest that can be used to detect a particular protein of interest, for example using an antibody. The method for producing polyclonal and/or monoclonal antibodies that specifically bind to polypeptides useful in the present invention is known to those of skill in the art and may be found in, for example, Dymecki, et al., (J. Biol. Chem. 267:4815, 1992); Boersma and Van Leeuwen, (J. Neurosci. Methods 51:317, 1994); Green, et al., (Cell 28:477, 1982); and Arnheiter, et al., (Nature 294:278, 1981). In one embodiment, an immunoassay can be used to quantitate the levels of proteins in cell samples. The invention is not limited to a particular assay procedure, and therefore, is intended to include both homogeneous and heterogeneous procedures.

Exemplary immunoassays that may be conducted according to the invention include fluorescence polarization immunoassay (FPIA)₅ fluorescence immunoassay (FIA), enzyme immunoassay (EIA), nephelometric inhibition immunoassay (NIA), enzyme-linked immunosorbent assay (ELISA), and radioimmunoassay (RIA). An indicator moiety, or label group, may be attached to the subject antibodies and is selected so as to meet the needs of various uses of the method that are often dictated by the availability of assay equipment and compatible immunoassay procedures. General techniques to be used in performing the various immunoassays noted above are known to those of ordinary skill in the art.

Alternatively other methods can be used such as Western blot analysis that includes electrophoretically separating proteins on a polyacrylamide gel, and after staining the separated proteins, the relative amount of each protein can be quantified by assessing its optical density. Alternatively, other methods such as dot-blot assays, FACS or immunohistochemistry can be used.

Typically, antibodies generated against the biomarkers of the disclosure can be used for visualizing for the presence of a protein of interest can be labeled, for example, using a reporter molecule such as fluorophores, enzymes, biotin, chemiluminescent molecules, bioluminescent molecules, digoxigenin, avidin, streptavidin or radioisotopes.

In yet another embodiment, the invention contemplates using a panel of antibodies that are generated against the marker polypeptides described herein.

### Data analysis

To facilitate the sample analysis operation, the data obtained by the reader from the device may be analyzed using a digital computer. Typically, the computer will be appropriately programmed for receipt and storage of the data from the device, as well as for analysis and reporting of the data gathered, for example, subtraction of the background, verifying that controls have performed properly, normalizing the signals, interpreting fluorescence data to determine the amount of hybridized target, normalization of background, and the like.

### Kits

The disclosure further provides kits for determining the expression level of the biomarkers described herein. The kits may be useful for determining who will benefit from treatment with a Hedgehog signaling inhibitor. A kit can comprise probes of genes identified in Table 1 can be used to measure gene expression of a test sample. For example, the kit comprises a computer readable medium which includes expression profile analysis software capable of being loaded into the memory of a computer system and which can convert the measured expression values into a risk score. A kit may further comprise nucleic acid controls, buffers, and instructions for use.

### Administration

The hedgehog signaling inhibitors described herein can be selectively administered in therapeutically effective amounts via any of the usual and acceptable modes known in the art, either singly or in combination with one or more therapeutic agents based on the individual having been determined to have an activated hedgehog signaling pathway as described herein. A therapeutically effective amount may vary widely depending on the severity of the disease, the age and relative health of the subject, the potency of the compound used and other factors.

### Examples

### Example 1

The multi-gene Hedgehog signature was initially developed by analyzing 40 FFPE medulloblastoma (MB) specimens. The matching fresh frozen specimens from the same cases were previously profiled as described by Y-J Cho and colleagues (Y-J Cho et al, JCO, 2010) and each individual case was determined to be either Hedgehog-activated or non-Hedgehog-activated based on its gene expression profile. A panel of 32 candidate genes that are differentially expressed in hedgehog positive (Hh+) versus hedgehog negative (Hh-) tumors and another 22 potential normalization genes were selected from a combined dataset of available profiling studies. All candidate genes that demonstrated differential Hh+ versus Hh- expression were consistent in all profiling studies evaluated. The RT-PCR assays for these candidate genes was developed and optimized for use in FFPE specimens. Candidate genes that did not show robust expression in FFPE specimen type were eliminated from further use. Assays with robust performance in FFPE for 10 up-regulated and 8 down-regulated genes in Hedgehog+ versus Hedgehog- tumors plus 5 control genes, were further selected and assembled onto a TLDA card which offers more economic tissue consumption compared to the single assay format. This panel of 23 candidate genes was then analyzed in the 40 FFPE specimens with known Hedgehog activation status.

The optimal model that has the minimal error in predicting the Hedgehog activation status and involves the least number of genes was selected using the Elastic Net method (J. Friedman, T. Hastie, and R. Tibshirani, J. of Statistical Software, 2008). Two optimal models, one with a 5-gene signature and the other with an 8-gene signature, showed similar performance in a 5-fold cross validation. These models allow computation of a probability score of being Hedgehog+ for a given sample based on the expression levels of either 5 or 8 genes, respectively. A cut-off was established based on the probability score to make determination of the Hedgehog+ versus Hedgehog- status for tested samples.

In addition, it was explored to use one single gene (SPHK1, OTX2, SFRP1, PDLIM3, or SHROOM2) to make the prediction of the Hedgehog activation status and a cut-off for each of these genes for making Hedgehog+ versus Hedgehog- calls was established using this set of 40 medulloblastoma tumors.

Both the 5- and 8-gene models as well as the single gene models were further externally validated in an independent sample set of 25 medulloblastoma tumors. All 25 patients had pathologically confirmed diagnosis including 13 with classic histology, 9 with nodular/desmoplastic histology, and 2 with large cell/anaplastic histology. Among the 25 patients, 13 were male and 12 female. Tissue specimens from 24 patients were collected at diagnosis and one patient collected after chemotherapy treatment. The median age of these 25 patients at diagnosis is 3 years-old with a range between 6 months and 16 years. The FFPE specimens from these 25 medulloblastoma cases were analyzed by the method of the invention to determine the Hedgehog activation status by the 5-gene and the 8-gene models. The matching fresh frozen tumor tissue specimens from the same 25 patients collected at the same time points as the FFPE samples were subjected to gene expression profiling using the GeneChip human genome U133 Plus 2.0 array (Affymetric, Santa Clara, CA). The 25 patients were classified to Subgroup c3 (Hedgehog activated sub-class) or non-Subgroup c3 tumors based on gene profile-based molecular sub-classification of medullobastoma as described by Y-J Cho and his colleagues (YJ Cho et al. JCO, 2010).

Eight out of the 25 patients were determined to have a Subgroup c3, Hedgehog-activated tumor, while 17 with non-Hedgehog-activated tumors. This molecular classification of the 25 medulloblastoma patients was performed prior to the analysis of the FFPE tumor specimens by the method of the invention. Therefore, the FFPE specimens that were analyzed by the method of the invention were considered to have known Hedgehog activation status. Based on the expression levels of the 8 genes (GLI-1, OTX-2, SHROOM2, PDLIM3, SPHK1, SFRP1, APBA2 and SPATA20), a predictive model was used to compute a probability score of being Hedgehog+ (0-100%) for a given sample. Based on a pre-specified probability cut-off, each specimen was determined to be either Hedgehog-activated or non-Hedgehog-activated. Eight specimens were called Hedgehog-activated and the remaining 17 were called non-Hedgehog-activated, demonstrating 100% agreement with the known Hedgehog activation status of these samples. In addition, the median probability score for 17 non-Hedgehog-activated tumors is 0.9% with a range between 0.2% and 3.1%. The median probability score for 8 Hedgehog-activated tumors is 87.0% ranging from 70.9% to 96.6%. The considerable difference in the median probability score between the negative and positive cases suggested the robustness of the Hedgehog+ versus Hedgehog- determination by the 8-gene model.

Based on the expression levels of the 5 genes (GLI-1, OTX-2, SHROOM2, PDLIM3, SPHK1) and the associated predictive model, a probability score was calculated for each of the 25 specimens and compared to a pre-specified cut-off. Hedgehog activation status was then determined for each tumor, again in 100% agreement with the known Hedgehog activation status. The median probability score for the 17 non-Hedgehog-activated tumors is 0.7% ranging from 0.1% to 3.0% and for 8 Hedgehog-activated tumors 87.9% ranging from 69.1 % to 97.6%, demonstrating the similar robustness of Hedgehog status determination as the 5-gene model.

Based on the expression level of a single gene, SPHK1, OTX2, SFRP1, PDLIM3, or SHROOM2, a Hedgehog+ or Hedgehog- call was made to the 25 medulloblastoma cases based on a pre-specified threshold for each of the genes. The Hedgehog calls made by SPHK1, OTX2, or SFRP1 for each of the 25 tumors were in complete agreement with the known Hedgehog activation status with 8 Hedgehog+ calls and 17 Hedgehog- calls. Based on PDLIM3, 24/25 correct calls were made. One Hedgehog+ tumor was wrongly called Hedgehog-, resulting in 87.5% sensitivity and 100% specificity of the prediction. The prediction made based on SHROOM2 demonstrated 75% sensitivity and 94% specificity with 22/25 correct calls. Two Hedgehog+ tumors were wrongly called Hedgehog- and one Hedgehog- tumor called Hedgehog+. Therefore, in addition to the multi-gene models that rely on composite measurement of involved genes, single genes within the multi-gene Hedgehog signature demonstrated significant predictive power by itself in determining Hedgehog activation status for medulloblastoma. The present study provides a validation that each of these genes in the method of the invention can be used to determine Hedgehog activation status in medulloblastoma.

The control genes HUWE1, LAPR1, SOD1 and YME1L1 were selected to normalize the expression of the genes that classified the medulloblastoma (MB) tumors based on hedgehog activation status. Ideally, the control gene should be expressed stably and at a similar level in all tumor tissues under investigation. Several studies have suggested that even widely used control genes such as β-actin and GAPDH are unsuitable in certain situations. In addition, in a combined dataset from available external profiling studies of fresh frozen MB tumor specimens these widely used control genes showed a very high expression levels of greater than lg2>11 as compared to the hedgehog pathway genes with expression levels around lg2>8.. Thus the controls genes with similar expression levels as the hedgehog pathway genes were selected. Any probesets that had an expression level of lg2<8 were not successfully detected by RT-PCR analysis in our study.

An important criterion for the selection of the controls is primarily based on the invariance of the genes across all the data sets analyzed. The percent coefficient of variance was set to be less than 4 percent. The normalization genes were further selected based on whether it is feasible to design an assay with <80 bp amplicon size and then whether robust expression could be demonstrated in FFPE samples with the optimized assay.

Exemplary probes are available as shown in Table 3.

**Table 3**

| Gene | ABI Assay ID | ReqSeq | exon boundary | Function in Signature |
|---|---|---|---|---|
| GLI1 | Hs00171790_m1 | NM_005269.2 | 11 and 12 | Up |
| PDLIM3 | Hs01062534_m1 | NM_014476.3 | 6 and 7 | Up |
| SHROOM2 | Hs01113636_m1 | NM_001649.2 | 9 and 10 | Up |
| SPHK1 | Hs00184211_m1 | NM_182965.2 | 5 and 6 | Up |
| OTX2 | Hs00222238_m1 | NM_021728.2 | 4 and 5 | Down |
| APBA2 | Hs01125385_m1 | NM_005503.3 | 8 and 9 | Up |
| HUWE1 | Hs00948075_m1 | NM_031407.4 | 67 and 68 | Control |
| YME1L1 | Hs00204609_m1 | NM_139312.1 | 1 and 2 | Control |
| SOD1 | Hs00533490_m1 | NM_000454.4 | 1 and 2 | Control |
| LARP1 | Hs00391726_m1 | NM_015315.3 | 3 and 4 | Control |

Exemplary probes for SFRP1 include a forward primer 5'-CCAATGCCACCGAAGCC-3' (SEQ ID NO:1) and reverse primer 5-TCACAGGGAGGACACACCG-3' (SEQ ID NO:2) and FAM. Exemplary probes for SPATA20 include a forward primer 5'-CAAGGCCAGGAAGGAAAACA-3' (SEQ ID NO:3) and reverse primer 5'-CACCAGTGGCAGGTGGAGTA3' (SEQ ID NO:4) and FAM.

### Example 2

Adult cancer patients including patients with medulloblastoma, were enrolled into an ongoing phase I, dose escalation trial of methyl-4'-trifluoromethoxy-biphenyl-3-carboxylic acid [6-(cis-2,6-dimethyl-morpholin-4-yl)-pyridin-3-yl]-amide to evaluate safety and tolerability of methyl-4'-trifluoromethoxy-biphenyl-3-carboxylic acid [6-(cis-2,6-dimethyl-morpholin-4-yl)-pyridin-3-yl]-amide and assess maximally tolerated dose of methyl-4'-trifluoromethoxy-biphenyl-3-carboxylic acid [6-(cis-2,6-dimethyl-morpholin-4-yl)-pyridin-3-yl]-amide for adult patients. Archival FFPE tumor specimens collected prior to the start of the trial are available for analysis by the method of the invention from 3 enrolled medulloblastoma patients in this trial. One patient treated with a daily dose of 200 mg of methyl-4'-triftuoromethoxy-biphenyl-3-carboxylic acid [6-(cis-2,6-dimethyl-morpholin-4-yl)-pyridin-3-yl]-amide achieved a partial response (PR) by the RECIST criteria after 2 months of treatment and the response maintained for 4 months before disease progression. The other metastatic medulloblastoma patient without a measurable lesion was treated with a 1500 mg daily dose and demonstrated metabolic partial response measured by positron emission tomography (PET) which lasted for 7 months before disease relapse. The 3^{rd} patient progressed rapidly after only 52 days of methyl-4'-trifluoromethoxy-biphenyl-3-carboxylic acid [6-(cis-2,6-dimethyl-morpholin-4-yl)-pyridin-3-yl]-amide treatment at a daily dose of 200 mg.

Based on the 8-gene and 5-gene predictive model, the method of the invention was used to determine the Hedgehog activation status in tumor for these 3 patients. The two responders were determined to have Hedgehog-activated tumor while the tumor from the non-responder was called non-Hedgehog-activated. In addition, the single genes, SPHK1, OTX2, SFRP1, PDLIM3, or SHROOM2 were each used to determine the Hedgehog activation status based on the pre-established cut-off for each of the genes. By each of the single gene, the same Hedgehog-activated and non-Hedgehog-activated calls were made to the two responders and one non-responder, respectively. Among the 3 patients, only one patient had adequate tumor tissue samples available for additional mutational analysis of the PTCH1 and SMO gene. This patient who achieved metabolic partial response and was called Hedgehog+ by the method of the invention was found to have a somatic mutation in PTCH1 gene. Inactivating PTCH1 mutations have been identified as a major mechanism of constitutively activating Hedgehog pathway in medulloblastoma. Therefore, identification of this PTCH1 mutation provides a mechanistic basis for Hedgehog pathway activation and observed clinical activity against Hedgehog signaling inhibitor in this patient and further validates the finding by the method of the invention.

### Example 3

The method of the invention was used to verify the Hedgehog activation status for another panel of 40 medulloblastomas with pathologically confirmed diagnosis. Among the 40 cases, 26 had classic medulloblastoma histology, 12 nodular/desmoplastic histology, and 1 large cell/anaplastic histology. Other demographic data associated with these cases were not available. The Hedgehog activation status of these tumors was previously characterized by the affymetric gene expression profiling method as described by Y-J Cho and his colleagues (YJ Cho et al. JCO, 2010). The Hedgehog-activated call in 15 cases and the non-Hedgehog-activated call in the remaining 25 cases were verified in FFPE tumor specimens by the method of the invention based on the 8-gene model, the 5-gene model and each of the single genes SPHK1, OTX2, SFRP1, PDLIM3, or SHROOM2.

### Example 4

In addition to the Hedgehog-activated medulloblastoma, basal cell carcinoma (BCC) is another cancer type that is mostly driven by mutations or genetic lesions of the Hedgehog pathway genes that lead to constitutive activation of the Hedgehog pathway. Over 90% of the patients with BCC, the most prevalent skin cancer, were found to either have inactivating mutations in the PTCH1 gene or less frequently genetic lesions of other Hedgehog pathway genes such as activating mutations of SMO. In an ongoing phase I, dose escalation trial of methyl-4'-trifluoromethoxy-biphenyl-3-carboxylic acid [6-(cis-2,6-dimethyl-morpholin-4-yl)-pyridin-3-yl]-amide, an Hedgehog signaling inhibitor antagonizing SMO, adult BCC patients were enrolled among patients with other solid tumors. Mutational analysis of PTCH1 and SMO by directly sequencing was performed to the archival tumor specimens collected at the time of diagnosis from the enrolled BCC patients. Six BCC patients were found to have mutations in either PTCH or SMO, including two Gorlin Syndrome patients where a germline PTCH1 mutation was identified. The method of the invention was used to determine the Hedgehog activation status of BCC tumors from these patients. With both the 5-gene and 8-gene models, the probability score of all 6 cases were above the pre-specified cut-off and therefore all were called Hedgehog-activated. Among these 6 patients, 2 achieved confirmed partial response on methyl-4'-trifluoromethoxy-biphenyl-3-carboxylic acid [6-(cis-2,6-dimethyl-morpholin-4-yl)-pyridin-3-yl]-amide treatment, 2 had stable disease for more than 6 month, and the other 2 patients discontinued methyl-4'-trifluoromethoxy-biphenyl-3-carboxylic acid [6-(cis-2,6-dimethyl-morpholin-4-yl)-pyridin-3-yl]-amide due to adverse events after less than 2 months of therapy which might be too brief to observe any clinical activities. The consistency demonstrated in the positive mutation results in PTCH1/SMO and the Hedgehog+ calls made by the method of the invention, although in another cancer type, further validates the method of the invention. The method of the invention can be used for identifying other types of tumor with the same underlining Hedgehog-mutation driven etiology that might preferentially benefit from a Hedgehog signaling inhibitor therapy.

### Example 5

Immunohistochemistry (IHC) analysis of GLI-1 and SFRP-1 expression at the protein level was described in PA Northcott et al., JCO, 2010). It was suggested that the presence of the positive IHC staining from these two protein markers could be used as a mean of identifying Hh+ medulloblastoma. GLI-1 and SFRP-1 are both individual genes described in the method of invention. Using antibodies against the protein product of GLI-1 (Cell Signaling, Beverly, MA) and SFRP-1 (Abcam, Cambridge, MA), 40 medulloblastoma FFPE tissue specimens with Hh activation status determined by the method of invention were subjected to the IHC analysis. Specimens with any levels of either nuclear staining of Gli-1 or cytoplasmic staining of SFRP-1 within the tumor content on slides were called Hh positive. Specimens with no staining from both markers in the appropriate cellular location were called Hh negative. The Hh activation status determined by the IHC method and by the method of invention was consistent in all cases tested, resulting in 100% agreement between the two methods. In addition, high degree of concordance between the Gli-1 and SFRP-1 IHC staining was observed. This data not only further validated the method of invention but also confirm that the individual markers described in the method of invention can be expressed not only at the transcript levels but also at the protein level.

## Claims

1. A method of analyzing a biological sample of a subject with cancer, comprising determining a level of expression of all of the biomarkers GLI-1, OTX-2, SHROOM2, PDLIM3, and SPHK1 in the biological sample taken from the subject, wherein the level of expression of the biomarkers in comparison to a control provide a diagnostic indicator of whether the subject has an increased likelihood of response to a methyl-4'-trifluoromethoxy-biphenyl-3-carboxylic acid [6-(cis-2,6-dimethyl-morpholin-4-yl)-pyridin-3-yl]-amide or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol.

2. A method of selecting a subject having cancer for treatment with a Hedgehog signaling inhibitor, the method comprising determining the level of expression of at least five biomarkers selected from the group consisting of GLI-1, OTX-2, SHROOM2, PDLIM3, SPHK1, SFRP1, APBA2 and SPATA20, in a biological sample derived from the subject, thereby to predict an increased likelihood of response to a Hedgehog signaling inhibitor.

3. A method of selecting a subject having cancer for treatment with methyl-4'-trifluoromethoxy-biphenyl-3-carboxylic acid [6-(cis-2,6-dimethyl-morpholin-4-yl)-pyridin-3-yl]-amide or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol, the method comprising determining the level of SHROOM2 in a biological sample derived from the subject, thereby to predict an increased likelihood of response to methyl-4'-trifluoromethoxy-biphenyl-3-carboxylic acid [6-(cis-2,6-dimethyl-morpholin-4-yl)-pyridin-3-yl]-amide or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol.

4. A method of selecting a subject having cancer for treatment with methyl-4'-trifluoromethoxy-biphenyl-3-carboxylic acid [6-(cis-2,6-dimethyl-morpholin-4-yl)-pyridin-3-yl]-amide or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol, the method comprising determining the level of SPHK1 in a biological sample derived from the subject, thereby to predict an increased likelihood of response to methyl-4'-trifluoromethoxy-biphenyl-3-carboxylic acid [6-(cis-2,6-dimethyl-morpholin-4-yl)-pyridin-3-yl]-amide or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol.

5. The method according to any one of claims 2 to 4, wherein the hedgehog signaling inhibitor is methyl-4'-trifluoromethoxy-biphenyl-3-carboxylic acid [6-(cis-2,6-dimethyl-morpholin-4-yl)-pyridin-3-yl]-amide or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol.

6. The method according to any one of claims 1-5, wherein the level of expression of mRNA is determined.

7. A Hedgehog signaling inhibitor, wherein the inhibitor is Jervine, GANT61, purmorphamine, SAG, SANT-2, tomatidine, zerumbone, GDC-0449, XL139, IPI926, IPI609 (IPI269609), BMS-833923/XL139, TAK-441, PF-04449913, methyl-4'-trifluoromethoxy-biphenyl-3-carboxylic acid [6-(cis-2,6-dimethyl-morpholin-4-yl)-pyridin-3-yl]-amide, or 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol, for use in a method of treating cancer in a patient in need thereof, comprising selectively administering the Hedgehog signaling inhibitor to the patient on the basis of the patient has an elevated level of expression of the biomarkers GLI-1, SHROOM2, PDLIM3, and SPHK1 and a decreased level of expression of the biomarker OTX-2.

8. The inhibitor for use according to claim 7, wherein a sample from the patient is assayed for the expression of the biomarkers and the sample is a Formalin-Fixed, Paraffin-Embedded tissue (FFPE).

9. The method according to any one of claims 1-6, or the inhibitor for use according to claim 7 or 8, wherein the cancer is BCC, medulloblastoma, rhabdomyosarcoma, CML, bone sarcoma, soft tissue sarcoma, pancreatic cancer, small cell lung cancer, prostate cancer Gorlin syndrome, or breast cancer.

10. The method according to any one of claims 1-6, or the inhibitor for use according to claim 7 or 8, wherein the cancer is medulloblastoma.

11. The method according to any one of claims 1-6, or according to claim 9 or claim 10, or the inhibitor for use according to claim 7 or 8, wherein the inhibitor is methyl-4'-trifluoromethoxy-biphenyl-3-carboxylic acid [6-(cis-2,6-dimethyl-morpholin-4-yl)-pyridin-3-yl]-amide.

12. The method according to any one of claims 1-6, or according to claim 9 or claim 10, or the inhibitor for use according to claim 7 or 8, wherein the inhibitor is 2-[(R)-4-(6-benzyl-4,5-dimethyl-pyridazin-3-yl)-2-methyl-3,4,5,6-tetrahydro-2H-[1,2']bipyrazinyl-5'-yl]-propan-2-ol.

## Patentansprüche

1. Verfahren zur Analyse einer biologischen Probe eines Individuums, bei dem eine Krebserkrankung vorliegt, umfassend Bestimmen eines jeweiligen Expressionsniveaus der Biomarker GLI-1, OTX-2, SHROOM2, PDLIM3 und SPHK1 in der dem Individuum entnommenen biologischen Probe, wobei mit dem Expressionsniveau der Biomarker im Vergleich mit einer Kontrolle ein diagnostischer Indikator dafür bereitgestellt wird, ob bei dem Individuum eine erhöhte Wahrscheinlichkeit eines Ansprechens auf ein Methyl-4'-trifluor-methoxybiphenyl-3-carbonsäure-[6-(cis-2,6-dimethylmorpholin-4-yl)pyridin-3-yl]amid oder 2-[(R)-4-(6-Benzyl-4,5-dimethylpyridazin-3-yl)-2-methyl-3,4,5,6-tetrahydro-2H-[1,2']bipyrazinyl-5'-yl]propan-2-ol besteht.

2. Verfahren zum Auswählen eines Individuums mit einer Krebserkrankung für eine Behandlung mit einem Hedgehog-Signalgebung-Inhibitor, wobei das Verfahren Bestimmen des Expressionsniveaus von wenigstens fünf aus der aus GLI-1, OTX-2, SHROOM2, PDLIM3, SPHK1, SFRP1, APBA2 und SPATA20 bestehenden Gruppe ausgewählten Biomarkern in einer von dem Individuum stammenden biologischen Probe umfasst, um somit eine erhöhte Wahrscheinlichkeit eines Ansprechens auf einen Hedgehog-Signalgebung-Inhibitor vorherzusagen.

3. Verfahren zum Auswählen eines Individuums mit einer Krebserkrankung für eine Behandlung mit Methyl-4'-trifluormethoxybiphenyl-3-carbonsäure-[6-(cis-2,6-dimethylmorpholin-4-yl)pyridin-3-yl]amid oder 2-[(R)-4-(6-Benzyl-4,5-dimethylpyridazin-3-yl)-2-methyl-3,4,5,6-tetrahydro-2H-[1,2']bipyrazinyl-5'-yl]propan-2-ol, wobei das Verfahren Bestimmen des Spiegels von SHROOM2 in einer von dem Individuum stammenden biologischen Probe umfasst, um somit eine erhöhte Wahrscheinlichkeit eines Ansprechens auf Methyl-4'-trifluormethoxybiphenyl-3-carbonsäure-[6-(cis-2,6-dimethylmorpholin-4-yl)-pyridin-3-yl]amid bzw. 2-[(R)-4-(6-Benzyl-4,5-dimethylpyridazin-3-yl)-2-methyl-3,4,5,6-tetrahydro-2H-[1,2']bipyrazinyl-5'-yl]propan-2-ol vorherzusagen.

4. Verfahren zum Auswählen eines Individuums mit einer Krebserkrankung für eine Behandlung mit Methyl-4'-trifluormethoxybiphenyl-3-carbonsäure-[6-(cis-2,6-dimethylmorpholin-4-yl)pyridin-3-yl]amid oder 2-[(R)-4-(6-Benzyl-4,5-dimethylpyridazin-3-yl)-2-methyl-3,4,5,6-tetrahydro-2H-[1,2']bipyrazinyl-5'-yl]propan-2-ol, wobei das Verfahren Bestimmen des Spiegels von SPHK1 in einer von dem Individuum stammenden biologischen Probe umfasst, um somit eine erhöhte Wahrscheinlichkeit eines Ansprechens auf Methyl-4'-trifluor-methoxybiphenyl-3-carbonsäure-[6-(cis-2,6-dimethylmorpholin-4-yl)pyridin-3-yl]amid bzw. 2-[(R)-4-(6-Benzyl-4,5-dimethylpyridazin-3-yl)-2-methyl-3,4,5,6-tetrahydro-2H-[1,2']bipyrazinyl-5'-yl]propan-2-ol vorherzusagen.

5. Verfahren nach einem der Ansprüche 2 bis 4, wobei es sich bei dem Hedgehog-Signalgebung-Inhibitor um Methyl-4'-trifluormethoxybiphenyl-3-carbonsäure-[6-(cis-2,6-dimethylmorpholin-4-yl)pyridin-3-yl]amid oder 2-[(R)-4-(6-Benzyl-4,5-dimethylpyridazin-3-yl)-2-methyl-3,4,5,6-tetrahydro-2H-[1,2']bipyrazinyl-5'-yl]propan-2-ol handelt.

6. Verfahren nach einem der Ansprüche 1-5, wobei das mRNA-Expressionsniveau bestimmt wird.

7. Hedgehog-Signalgebung-Inhibitor, wobei es sich bei dem Inhibitor um Jervin, GANT61, Purmorphamin, SAG, SANT-2, Tomatidin, Zerumbon, GDC-0449, XL139, IPI926, IPI609 (IPI269609), BMS-833923/XL139, TAK-441, PF-04449913, Methyl-4'-trifluormethoxybiphenyl-3-carbonsäure-[6-(cis-2,6-dimethylmorpholin-4-yl)pyridin-3-yl]amid oder 2-[(R)-4-(6-Benzyl-4,5-dimethylpyridazin-3-yl)-2-methyl-3,4,5,6-tetrahydro-2H-[1,2']bipyrazinyl-5'-yl]propan-2-ol handelt, zur Verwendung bei einem Verfahren zur Krebsbehandlung bei einem dieser bedürfenden Patienten, wobei dem Patienten der Hedgehog-Signalgebung-Inhibitor auf der Grundlage, dass der Patient ein erhöhtes Expressionsniveau der Biomarker GLI-1, SHROOM2, PDLIM3 und SPHK1 und ein vermindertes Expressionsniveau des Biomarkers OTX-2 aufweist, selektiv verabreicht wird.

8. Inhibitor zur Verwendung nach Anspruch 7, wobei eine Probe aus dem Patienten auf die Expression der Biomarker getestet wird und es sich bei der Probe um ein FFPE(Formalin-Fixed, Paraffin-Embedded)-Gewebe handelt.

9. Verfahren nach einem der Ansprüche 1-6 oder Inhibitor zur Verwendung nach Anspruch 7 oder 8, wobei es sich bei der Krebserkrankung um BCC, Medulloblastom, Rhabdomyosarkom, CML, Knochensarkom, Weichgewebesarkom, Bauchspeicheldrüsenkrebs, kleinzelligen Lungenkrebs, Prostatakrebs, Gorlin-Syndrom oder Brustkrebs handelt.

10. Verfahren nach einem der Ansprüche 1-6 oder Inhibitor zur Verwendung nach Anspruch 7 oder 8, wobei es sich bei der Krebserkrankung um Medulloblastom handelt.

11. Verfahren nach einem der Ansprüche 1-6 oder nach Anspruch 9 oder Anspruch 10 oder Inhibitor zur Verwendung nach Anspruch 7 oder 8, wobei es sich bei dem Inhibitor um Methyl-4'-trifluormethoxybiphenyl-3-carbonsäure-[6-(cis-2,6-dimethylmorpholin-4-yl)pyridin-3-yl]amid handelt.

12. Verfahren nach einem der Ansprüche 1-6 oder nach Anspruch 9 oder Anspruch 10 oder Inhibitor zur Verwendung nach Anspruch 7 oder 8, wobei es sich bei dem Inhibitor um 2-[(R)-4-(6-Benzyl-4,5-dimethylpyridazin-3-yl)-2-methyl-3,4,5,6-tetrahydro-2H-[1,2']bipyrazinyl-5'-yl]propan-2-ol handelt.

## Revendications

1. Méthode d'analyse d'un échantillon biologique d'un sujet atteint de cancer, comprenant la détermination d'un niveau d'expression de l'ensemble des biomarqueurs GLI-1, OTX-2, SHROOM2, PDLIM3 et SPHK1 dans l'échantillon biologique prélevé chez le sujet, où le niveau d'expression des biomarqueurs par comparaison à un témoin fournit un indicateur de diagnostic permettant de déterminer si le sujet présente une probabilité supérieure de réponse au [6-(cis-2,6-diméthyl-morpholin-4-yl)-pyridin-3-yl]-amide d'acide méthyl-4'-trifluorométhoxy-biphényl-3-carboxylique ou au 2-[(R)-4-(6-benzyl-4,5-diméthyl-pyridazin-3-yl)-2-méthyl-3,4,5,6-tétrahydro-2H-[1,2']bipyrazinyl-5'-yl]-propan-2-ol.

2. Méthode de sélection d'un sujet atteint de cancer pour traitement par un inhibiteur de la voie de signalement Hedgehog, la méthode comprenant la détermination du niveau d'expression d'au moins cinq biomarqueurs choisis dans le groupe constitué par GLI-1, OTX-2, SHROOM2, PDLIM3, SPHK1, SFRP1, APBA2 et SPATA20, dans un échantillon biologique dérivé du sujet, ce qui permet de prédire une probabilité supérieure de réponse à un inhibiteur de la voie de signalement Hedgehog.

3. Méthode de sélection d'un sujet atteint de cancer pour traitement par [6-(cis-2,6-diméthyl-morpholin-4-yl)-pyridin-3-yl]-amide d'acide méthyl-4'-trifluorométhoxy-biphényl-3-carboxylique ou 2-[(R)-4-(6-benzyl-4,5-diméthyl-pyridazin-3-yl)-2-méthyl-3,4,5,6-tétrahydro-2H-[1,2']bipyrazinyl-5'-yl]-propan-2-ol, la méthode comprenant la détermination du niveau de SHROOM2 dans un échantillon biologique dérivé du sujet, ce qui permet de prédire une augmentation de la probabilité de réponse au [6-(cis-2,6-diméthyl-morpholin-4-yl)-pyridin-3-yl]-amide d'acide méthyl-4'-trifluorométhoxy-biphényl-3-carboxylique ou au 2-[(R)-4-(6-benzyl-4,5-diméthyl-pyridazin-3-yl)-2-méthyl-3,4,5,6-tétrahydro-2H-[1,2']bipyrazinyl-5'-yl]-propan-2-ol.

4. Méthode de sélection d'un sujet atteint de cancer pour traitement par [6-(cis-2,6-diméthyl-morpholin-4-yl)-pyridin-3-yl]-amide d'acide méthyl-4'-trifluorométhoxy-biphényl-3-carboxylique ou 2-[(R)-4-(6-benzyl-4,5-diméthyl-pyridazin-3-yl)-2-méthyl-3,4,5,6-tétrahydro-2H-[1,2']bipyrazinyl-5'-yl]-propan-2-ol, la méthode comprenant la détermination du niveau de SPHK1 dans un échantillon biologique dérivé du sujet, ce qui permet de prédire une augmentation de la probabilité de réponse au [6-(cis-2,6-diméthyl-morpholin-4-yl)-pyridin-3-yl]-amide d'acide méthyl-4'-trifluorométhoxy-biphényl-3-carboxylique ou au 2-[(R)-4-(6-benzyl-4,5-diméthyl-pyridazin-3-yl)-2-méthyl-3,4,5,6-tétrahydro-2H-[1,2']bipyrazinyl-5'-yl]-propan-2-ol.

5. Méthode selon l'une quelconque des revendications 2 à 4, où l'inhibiteur de la voie de signalement Hedgehog est le [6-(cis-2,6-diméthyl-morpholin-4-yl)-pyridin-3-yl]-amide d'acide méthyl-4'-trifluorométhoxy-biphényl-3-carboxylique ou le 2-[(R)-4-(6-benzyl-4,5-diméthyl-pyridazin-3-yl)-2-méthyl-3,4,5,6-tétrahydro-2H-[1,2']bipyrazinyl-5'-yl]-propan-2-ol.

6. Méthode selon l'une quelconque des revendications 1 à 5, où le niveau d'expression de l'ARNm est déterminé.

7. Inhibiteur de la voie de signalement Hedgehog, où l'inhibiteur est l'un des suivants : Jervine, GANT61, purmorphamine, SAG, SANT-2, tomatidine, zerumbone, GDC-0449, XL139, IPI926, IPI609 (IPI269609), BMS-833923/XL139, TAK-441, PF-04449913, [6-(cis-2,6-diméthyl-morpholin-4-yl)-pyridin-3-yl]-amide d'acide méthyl-4'-trifluorométhoxy-biphényl-3-carboxylique ou 2-[(R)-4-(6-benzyl-4,5-diméthyl-pyridazin-3-yl)-2-méthyl-3,4,5,6-tétrahydro-2H-[1,2']bipyrazinyl-5'-yl]-propan-2-ol, pour utilisation dans une méthode de traitement du cancer chez un patient le nécessitant, comprenant l'administration sélective de l'inhibiteur de signalement Hedgehog au patient sur la base d'un niveau élevé d'expression des biomarqueurs GLI-1, SHROOM2, PDLIM3 et SPHK1, et d'une diminution du niveau d'expression du biomarqueur OTX-2.

8. Inhibiteur pour utilisation selon la revendication 7, où un échantillon issu du patient est dosé pour déterminer l'expression des biomarqueurs et l'échantillon est un tissu fixé sur formaline et intégré à de la paraffine (FFPE).

9. Méthode selon l'une quelconque des revendications 1 à 6, ou inhibiteur pour utilisation selon la revendication 7 ou 8, où le cancer est l'un des suivants : BCC, médulloblastome, rhabdomyosarcome, CML, sarcome osseux, sarcome des tissus mous, cancer pancréatique, cancer du poumon à petites cellules, cancer de la prostate, syndrome de Gorlin ou cancer du sein.

10. Méthode selon l'une quelconque des revendications 1 à 6, ou inhibiteur pour utilisation selon la revendication 7 ou 8, où le cancer est un médulloblastome.

11. Méthode selon l'une quelconque des revendications 1 à 6, ou selon la revendication 9 ou la revendication 10, ou inhibiteur pour utilisation selon la revendication 7 ou 8, où l'inhibiteur est le [6-(cis-2,6-diméthyl-morpholin-4-yl)-pyridin-3-yl]-amide d'acide méthyl-4'-trifluorométhoxy-biphényl-3-carboxylique.

12. Méthode selon l'une quelconque des revendications 1 à 6, ou selon la revendication 9 ou la revendication 10, ou inhibiteur pour utilisation selon la revendication 7 ou 8, où l'inhibiteur est le 2-[(R)-4-(6-benzyl-4,5-diméthyl-pyridazin-3-yl)-2-méthyl-3,4,5,6-tétrahydro-2H-[1,2']bipyrazinyl-5'-yl]-propan-2-ol.
